# EUROPEAN PATENT SPECIFICATION

(11) **EP 2 992 500 B1**
(45) Date of publication and mention of the grant of the patent: **26.12.2018**
(21) Application number: 14791330.5
(22) Date of filing: 01.05.2014
(51) Int. Cl.: G16H 40/63

(54) **A METHOD, DEVICE AND SYSTEM FOR HEALTHCARE DEVICE ADAPTATION**
VERFAHREN, GERÄT UND SYSTEM ZUR ANPASSUNG VON GESUNDHEITSGERÄTEN
PROCÉDÉ, DISPOSITIF ET SYSTÈME D'ADAPTATION DES DISPOSITIFS DE SOINS DE SANTÉ

(30) Priority: 02.05.2013 US 201361818850 P
(43) Date of publication of application: 09.03.2016
(73) Proprietor: Eloquence Communications, Inc., Ann Arbor, MI 48105 (US)
(72) Inventor: TRAUGHBER, Bryan James, Shaker Heights, Ohio 44120 (US); PATAK, Lance S., San Diego, California 92116 (US); DUGAN, Brendon, Johnson City, Tennessee 37601 (US); TEEGARDEN, Vaughn, Johnson City, Tennessee 37604 (US)
(74) Representative: Potter Clarkson LLP
(86) International application number: PCT/US2014/036343
(87) International publication number: WO 2014/179553

(56) References cited:
- US-A1- 2005 201 345
- US-A1- 2006 107 061
- US-A1- 2009 199 276
- US-A1- 2009 210 940
- US-A1- 2009 248 437
- US-A1- 2010 082 372
- US-A1- 2011 022 411
- US-A1- 2012 264 374

## Description

This invention was made with government support under Federal Grant Number 2R41MD006149-02 awarded by the National Institutes of Health, Institute for Minorities and Health Disparities. The government has certain rights in the invention.

### TECHNICAL FIELD

The present disclosure relates generally to healthcare systems and more particularly, but not exclusively, to systems and methods for providing a healthcare provider tracking system.

### BACKGROUND

In a healthcare environment, the ability for staff to quickly access information resources may be important for patient care and efficiency. With the proliferation of computerized systems, staff members are increasingly burdened by the need to remember multiple logins to access these systems. In addition to remembering logins, typing usernames and passwords slows down daily workflow and introduces unnecessary delay and frustration into the work process.

The introduction of more advanced nurse call systems, where logins are desirable to track who responds to patient requests, and where a high number of logins are required every day, means having a fast login method is desirable. Additionally, typing usernames and passwords requires physical contact with keyboard interfaces or touch-screens, which may act as vectors for infectious diseases in a healthcare environment. Document US2005/0201345 represents the closest prior art.

### Summary of the invention

The present invention discloses a method, device and system as defined by claims 1, 7 and 14.

### BRIEF DESCRIPTION OF THE DRAWINGS

The accompanying drawings, which are included as part of the present specification, illustrate one embodiment of the present invention and together with the general description given above and the detailed description of the preferred embodiment given below, serve to explain and teach the principles of the present invention.
Fig. 1 is an exemplary top-level drawing illustrating a healthcare provider tracking system.
Fig. 2 is an exemplary data flow diagram illustrating an embodiment of a data flow path between the user tag, patient device and server of Fig. 1.
Fig. 3 is an exemplary block diagram illustrating an embodiment of a method for healthcare provider tracking in accordance with an embodiment.
Fig. 4 is an exemplary block diagram illustrating an embodiment of a method for healthcare provider tracking in accordance with an embodiment.

It should be noted that the figures are not necessarily drawn to scale and that elements of similar structures or functions are generally represented by like reference numerals for illustrative purposes throughout the figures. It also should be noted that the figures are only intended to facilitate the description of the various embodiments.

### DETAILED DESCRIPTION

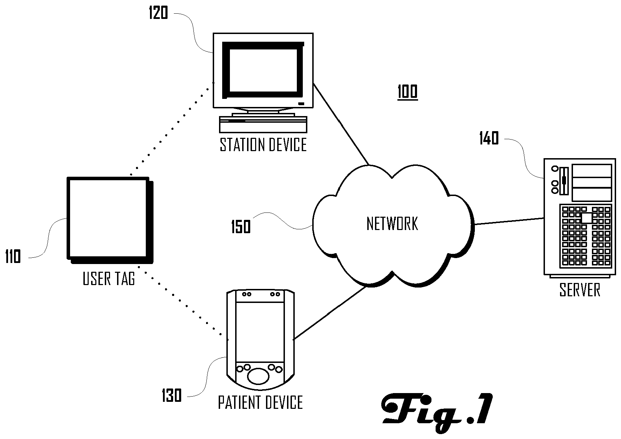

Turning to Fig. 1, the healthcare provider tracking system 100 is shown as including a station device 120, a patient device 130 and a server 140 that are operably connected via a network 150. A user tag 110 may also be operably connected with or communicate with the station device 120 or patient device 130.

The user tag 110 can be various types of devices that are operable to store and communicate data. For example, the user tag 110 can comprise a Near Field Communication ("NFC") device, a Radio Frequency Identification Device ("RFID"), a Bluetooth enabled device, a WiFi enabled device, or the like without limitation.

The devices 120 and 130 are depicted as desktop computer and tablet devices respectively, but in various embodiments, the devices 120 and 130 may be any suitable device including a smart phone, laptop computer, gaming device, server, or the like without limitation. In various embodiments, the user tag 110 can be a device operable to be wirelessly read, interrogated, and/or modified by a suitable device in close proximity to the user tag 110.

Additionally, the server 140 may be any suitable device or may comprise a plurality of devices, or may be a cloud-based system. In various embodiments, the network 150 may comprise one or more suitable wireless or wired network, including the Internet, a local-area network ("LAN"), a wide-area network ("WAN"), or the like.

In various embodiments, there may be a plurality of the devices 120, 130 and a plurality of user tags 110. For example, in an embodiment where the system 100 is implemented in a hospital, there may be a plurality of healthcare providers such as doctors, nurses and other healthcare staff that can carry a user tag 110. As described in more detail herein, these providers may use their user tag 110 to login and configure devices such as the station device 120 and patient device 130. A given device 120, 130 may be in a locked or limited functionality configuration, and when service providers approach a given device 120, 130, they can position their user tag 110 proximate to the device 120, 130 such that the device 120, 130 reads or obtains user data from the user tag 110, which allows the device 120, 130 to authenticate the service provider via the server 140, and configure the device 120, 130 for use by the authenticated service provider based on the given provider's access credentials defined by the server 140.

Station and patient devices 120, 130 are described herein for purposes of example only, and in some embodiments, various suitable devices in various suitable locations may be employed in a healthcare provider tracking system 100. Additionally, Fig. 1 depicts the user tag 110 in communication with both the station device 120 and patient device 130. Although embodiments may allow for simultaneous communication between a user tag 110 and a plurality of devices 120, 130, in various embodiments, the user tag 110 and user tag readers associated with a given device 120, 130 are only operable at relatively short distances (*e.g*., within about 0.1 cm, 0.5 cm, 1 cm, 2 cm, 5 cm, 10 cm, 50 cm, 100 cm, or the like). Accordingly, where devices 120, 130 are spaced apart at distances greater than this operable distance, the user tag 110 may therefore only communicate with only one device 120, 130 at a time.

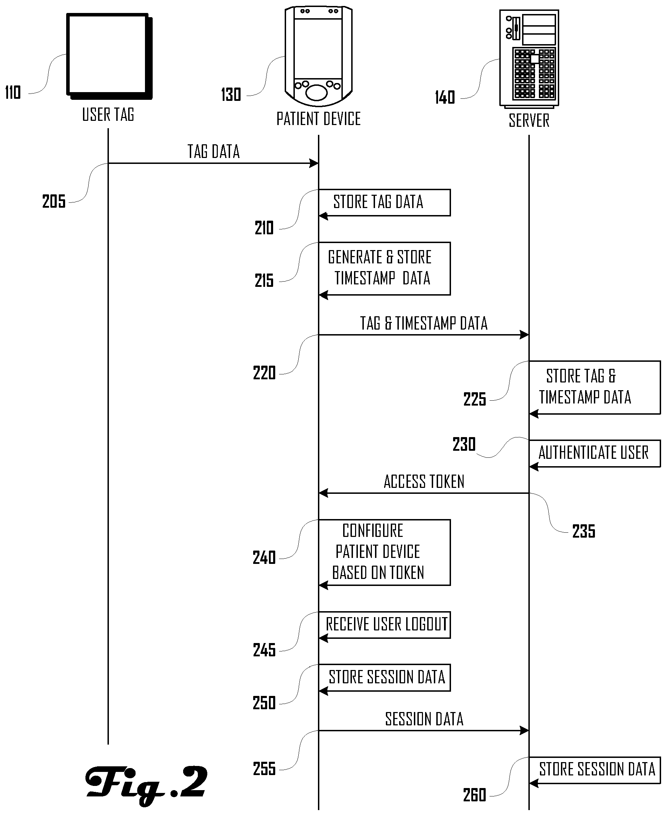

Fig. 2 is an exemplary data flow diagram illustrating an embodiment of a data flow path between the user tag 110, patient device 130 and server 140 of Fig. 1. The data flow begins where the user tag 110 sends tag data to the patient device, at 205. The patient device 130 stores tag data, at 210, and generates and stores a timestamp associated with the tag data, at 215. For example, in some embodiments, tag data may be used to login and configure the patient device 130, and tag data may comprise a tag ID, a user ID, a user password, a user token, user session data, user settings, profile data, or the like. Additionally, as described herein, it may be desirable to track when a user log into, or attempts to log into the patent device 130 for tracking purposes, and therefore a timestamp may be associated with the received tag data. In some embodiments, timestamps may be generated in relation to any suitable step in the processes or a user session as described herein.

Returning to the communications, tag data and timestamp data are sent to the server 140, at 220, and tag data and associated timestamp data are stored at the server 140, at 225. In an embodiment comprising a plurality of tags 110 and devices 120, 130 in a hospital, storing this timestamp data associated with the tag data may be desirable because it allows a system administrator to track healthcare providers performing services in and around the hospital. Such tracking may provide for provider accountability and promote increased efficiency and faster response time to patient requests and needs.

Returning again to the communications, the server 140 authenticates the user data based on the tag data, at 230, and sends an access token to the patient device 130, at 235. The patient device 130 is configured based on the access token, at 240. For example, authentication of a user may comprise comparing received tag data to authentication data stored at the server 140. In some embodiments, received tag data comprises a user ID and a tag ID, and both must correspond to an authentication entry on the server 140 for user authentication to occur. Each user tag 110 may have a unique serial number or identification number associated with the tag 110, and by requiring both a matching user ID and tag ID, fraudulent access attempts can be prevented where a malicious party attempts to re-program a user tag 110 with different user credentials or where an unauthorized user tag 110 is programmed with valid user credentials.

In various embodiments, it may be desirable to configure or reconfigure the patient device 130 when in use by different health care providers, health care staff, or others such as patients. For example, the patient device 130 may be configured for use by a patient (*e.g.,* as described in U.S. Patent Application 13/460,175), which provides a defined set of device functionalities via an interface. Such functionalities may include a patient scheduling orders or indicating various needs.

A nurse that is providing care or services to a patient may also use the patient device 130, but may configure the patient device interface to provide different functionalities by logging in with a user tag 110. Once authenticated, the patient device interface may be customized for the nurse user associated with the user tag 110. For example, the nurse user may have a user profile that includes favorites, user history, an access level, or other configuration settings.

Additionally, the interface may be configured to provide functionalities based on allowable user duties, user seniority, user status, or the like. For example, some health care staff may only be able to perform basic patient tasks such as providing comfort to and repositioning of a patient, providing food and drink, or attending to bathroom or hygiene needs. Such a staff member would therefore have a customized interface that provides functionalities related to these tasks but not others (*e.g*., as described in U.S. Patent Application 13/460,175). In contrast, a doctor may be authorized to change medication settings, provide a patient diagnosis, and perform certain medical procedures. Accordingly, a doctor may have a customized interface that provides functionalities related to these advanced tasks, whereas other users would not have access to such functionalities in their customized user interface.

Returning again to the communications, the patient device 130 receives a user logout, at 245, and stores session data at 250. Session data is sent to the server 140, at 255, and stored at 260. In various embodiments, it may be desirable to track and record actions performed by a user while the user is logged onto the patient device 140 (*i*.*e*., during a user session). While session data is depicted as being stored and sent to the server 140 after a user session, in some embodiments, session data may be communicated and stored in real-time or periodically during a user session.

In various embodiments, access to a customized user interface (via a patient device 130, station device 120, or the like) may provide a health care provider access to a task list, or may allow a health care provider to generate such a task list. A task list may allow the provider to respond to a patient, forward a patient request to another provider, send a message to another provider or send alert to the provider if a lapse in time has occurred that would warrant sending an alert to the provider, or send an alert at various programmed time intervals or at a programmed time, depending on the context of the patient request or task list item or provider preferences. Additionally, a task list may allow the provider to add, remove, edit, change the priority of, change the display order of, change the assignment of, or change an access setting of a task listed on a task list.

In further embodiments, a task list may be transferable. For example, if a given provider has a plurality of active tasks assigned to them on a task list, these active tasks may need to be re-assigned when the assigned provider goes on break, ends a shift, or otherwise is unavailable to perform the assigned task items according to defined criteria. In some embodiments, tasks may be re-assigned to a single provider or distributed among a plurality of providers. Such re-assignment may occur automatically without user interaction, or may be selectively performed by a healthcare provider, administrator, or the like.

In some embodiments, a customized user interface may allow for communication between and among a plurality of health care providers, patients or the like. For example, users may communicate via audio, text message, video, or the like.

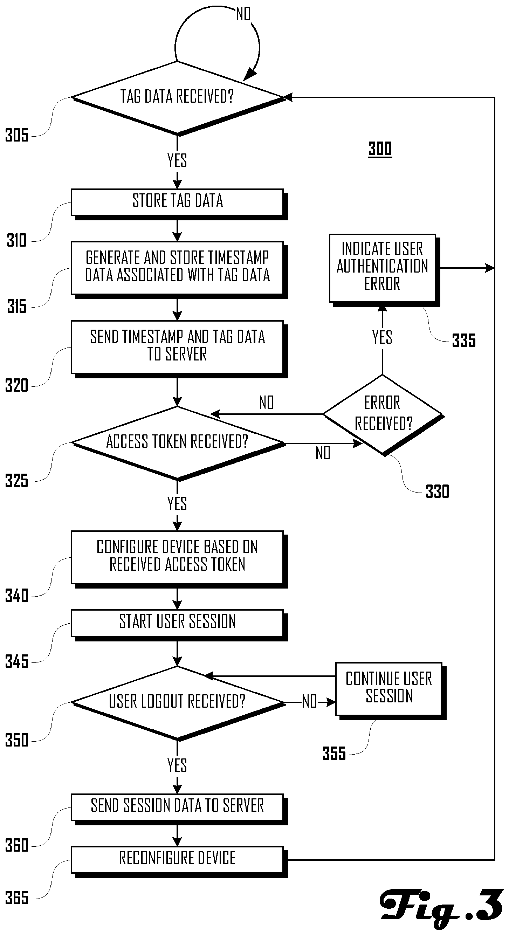

Fig. 3 is an exemplary block diagram illustrating an embodiment of a method 300 for healthcare provider tracking in accordance with an embodiment. The method 300 begins in decision block 305 where a determination is made whether tag data is received. If tag data is not received, the method 300 loops at block 305 until tag data is received. If tag data is received, the tag data is stored in block 310 and timestamp data is generated and stored in association with the tag data in block 315. In block 320, timestamp and tag data are sent to the server 140.

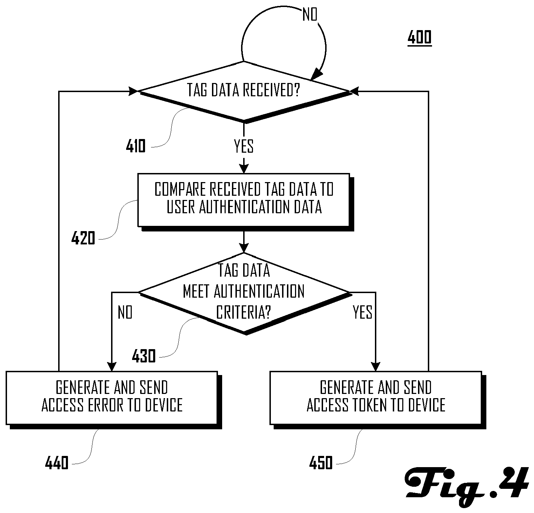

The method 300 continues to decision block 325 where a determination is made whether an access token is received. If an access token is not received, then in decision block 330, a determination is made whether an error message is received. If an error message is not received, the method 300 cycles back to decision block 325; however, if an error is received, then in block 335 a user authentication error is indicated, and the method 300 cycles back to decision block 305. For example, a nurse can tap his user tag 110 at a tag reader associated with a station or patient device 120, 130, and the obtained tag data can be sent to the server 140 for authentication. If the server 140 determines that the tag data does not meet authentication criteria (Fig. 4), the server may send an error indicating that the tag data does not meet authentication criteria.

Returning to Fig. 3, if an access token is received, then in block 340 the device is configured based on the received access token and a user session is initiated in block 345. For example, as discussed herein, a user interface on a patient device 130 may be customized, configured or reconfigured based on the received access token. The user interface may provide access to, obscure, or deactivate various functionalities of the patient device 130 or interface on the patient device 130. The user may perform functions on the device 130 with the modified or configured user interface during a user session, and then logout of the user session.

In decision block 350 a determination is made whether a user logout is received, and if not, the user session is continued in block 355 and the method 300 cycles back to decision block 350. If a user logout is received, then session data is sent to the server 140 in block 360, and the device 130 is reconfigured in block 365. The method 300 then cycles back to decision block 305. For example, as discussed herein, a device 120, 130 may comprise a user interface that is configured based on a received access token. When the user logs out of a user session, the user interface may then be reconfigured, which may be a configuration that the interface was in before the user session began. In some embodiments, a patient device 130 may be configured for patient use, and then configured for used by a nurse, doctor or other user when such a user logs onto the device 130 with a user tag 110. When the user logs out of a user session, the device may return to the patient configuration for patient use.

Similarly, a station device 120 may be a general-use device that can be accessed by medical providers and the station device 120 may be in a locked configuration or other default configuration. When such a user logs onto the station device 120 with a user tag 110, the station device 120 may be configured as discussed herein and then return to the default or locked configuration when the user logs out of a user session.

Fig. 4 is an exemplary block diagram illustrating an embodiment of a method 400 for healthcare provider tracking in accordance with an embodiment. The method 400 begins in decision block 410 where a determination is made whether tag data is received, and if not, the method 400 loops back to block 410. However, if tag data is received, then in block 420, received tag data is compared to user authentication data, and in decision block 430, a determination is made whether the received tag data meets authentication criteria.

For example, as discussed herein, tag data can comprise a tag identifier associated with the user tag 110 and can comprise an identifier associated with a user. The server 140 or other device may store data corresponding to user identifiers, tag identifiers, and relations therebetween. A determination whether received tag data meets authentication criteria may include a determination of whether a received user ID is valid and has required permissions; a determination whether a received tag ID is valid and has required permissions; and a determination whether the received user ID and tag ID are linked or associated.

Returning to the method 400, if tag data meets authentication criteria, then in block 450, an access token is generated and sent to the device that sent the tag data. The method 400 then cycles back to block 410. However, if received tag data does not meet authentication criteria, then in block 440 an access error message is generated and sent to the device that sent the tag data. The method 400 then cycles back to block 410.

It is understood that the embodiments described herein are for the purpose of elucidation and should not be considered limiting the subject matter of the present disclosure. Various modifications, uses, substitutions, combinations, and improvements, without departing from the scope of the present invention, would be evident to a person skilled in the art.

## Claims

1. A method of customizing a healthcare device (120, 130), the method comprising the healthcare device (120, 130) performing the following steps:
presenting a first user interface on the healthcare device configured for enabling a patient in a healthcare environment to enter a patient request;
receiving tag data from a user tag (110) associated with a selected healthcare provider in the healthcare environment comprising a healthcare provider identifier;
sending a portion of the tag data to an authentication server (140);
receiving an access token from the authentication server based on authentication of the portion of the tag data; and
presenting a second user interface on the healthcare device different from the first user interface, the second user interface configured for use by the selected healthcare provider based on the access token and the healthcare provider identifier to notify the selected healthcare provider of the patient request.

2. The method of claim 1, wherein the tag data is received from the user tag via near field communication.

3. The method of claim 1, further comprising:
receiving a logout via the second user interface; and
presenting the first user interface in place of the second user interface.

4. The method of claim 1, wherein the first user interface has functionalities for patient use, wherein the second user interface has functionalities for healthcare provider use, and wherein the second user interface and the healthcare provider functionalities are not accessible via the first user interface.

5. The method of claim 1, wherein receiving tag data occurs automatically without user interaction when a tag is present within operable range of a tag reader associated with the healthcare device.

6. The method of claim 1, wherein receiving the tag data includes:
generating a message; and
transmitting the generated message to a server operable to send one or more messages, based on the generated message, to one or more healthcare devices.

7. A healthcare device (120, 130) configured to:
present a first user interface configured for enabling a patient in a healthcare environment to enter a patient request;
receive tag data from a user tag (110) associated with a selected healthcare provider in the healthcare environment comprising a healthcare provider identifier;
send a portion of the tag data to an authentication server (140);
receive an access token from the authentication server based on authentication of the portion of the tag data; and
present a second user interface on the healthcare device different from the first user interface, the second user interface configured for use by the selected healthcare provider based on the access token and the healthcare provider identifier to notify the selected healthcare provider of the patient request.

8. The healthcare device of claim 7, wherein the first user interface of at least one selected healthcare device has functionalities for patient use, wherein the second user interface of the selected healthcare device has functionalities for healthcare provider use, and wherein the second user interface and the healthcare provider functionalities of the selected healthcare device are not accessible via the first user interface.

9. The healthcare device of claim 7, wherein the tag data further comprises a tag identifier, and wherein the access token is received from the authentication server based on a comparison of the tag identifier and the healthcare provider identifier to authentication data stored on the authentication server.

10. The healthcare device of claim 7, wherein the healthcare device is configured to:
generate session data based on the received tag data and actions performed via the second user interface; and
communicate the session data to the authentication server.

11. The healthcare device of claim 7, wherein obtaining the tag data from the user tag occurs automatically via near field communication without user interaction when the user tag is present within an operable range of a tag reader associated with the healthcare device.

12. The healthcare device of claim 7, wherein the healthcare device is configured to:
generate a message; and
transmit the message to the authentication server, wherein the authentication server is configured to send one or more messages to one or more healthcare devices.

13. The healthcare device of claim 7, wherein the user tag is configured to be carried by the selected healthcare provider associated with the user tag.

14. A system comprising the healthcare device (120, 130) of claim 7, the user tag (110) and the authentication server (140).

15. The system of claim 14, wherein the system comprises a first healthcare device and a second healthcare device, and wherein, for the second healthcare device, the first user interface is a locked interface, the second user interface has functionalities for healthcare provider use, and the second user interface and the healthcare provider functionalities are not accessible via the first user interface.

## Patentansprüche

1. Verfahren zur Anpassung eines Gesundheitsgeräts (120, 130),
wobei das Verfahren das Gesundheitsgerät (120, 130) umfasst und die folgenden Schritte durchführt:
Darstellen einer ersten Benutzerschnittstelle auf dem Gesundheitsgerät, das dazu ausgelegt ist, einem Patienten in einer Gesundheitsumgebung zu ermöglichen, eine Patientenanfrage einzugeben;
Empfangen von Tag-Daten von einem Benutzer-Tag (110), das mit einem ausgewählten Gesundheitsdienstleister in der Gesundheitsumgebung verknüpft ist, umfassend einen Gesundheitsdienstleisteridentifikator;
Senden eines Abschnitts der Tag-Daten an einen Authentifizierungsserver (140);
Empfangen eines Zugangstokens von dem Authentifizierungsserver basierend auf der Authentifizierung des Abschnitts der Tag-Daten; und
Darstellen einer zweiten Benutzerschnittstelle auf dem Gesundheitsgerät, die sich von der ersten Benutzerschnittstelle unterscheidet, wobei die zweite Benutzerschnittstelle zur Verwendung durch den ausgewählten Gesundheitsdienstleister basierend auf dem Zugangstoken und dem Gesundheitsdienstleisteridentifikator ausgelegt ist, um den ausgewählten Gesundheitsdienstleister der Patientenanfrage zu benachrichtigen.

2. Verfahren nach Anspruch 1, wobei die Tag-Daten von dem Benutzer-Tag über Nahfeldkommunikation empfangen werden.

3. Verfahren nach Anspruch 1, ferner umfassend:
Empfangen eines Log-outs über die zweite Benutzerschnittstelle; und Darstellen der ersten Benutzerschnittstelle anstelle der zweiten Benutzerschnittstelle.

4. Verfahren nach Anspruch 1, wobei die erste Benutzerschnittstelle Funktionalitäten zur Verwendung durch den Patienten aufweist, wobei die zweite Benutzerschnittstelle Funktionalitäten zur Verwendung durch den Gesundheitsdienstleister aufweist, und wobei die zweite Benutzerschnittstelle und die Gesundheitsdienstleisterfunktionalitäten nicht über die erste Benutzerschnittstelle zugänglich sind.

5. Verfahren nach Anspruch 1, wobei das Empfangen der Tag-Daten automatisch ohne Benutzerinteraktion erfolgt, wenn ein Tag in einem Bereich der Betriebsfähigkeit eines Tag-Lesegeräts vorhanden ist, das mit dem Gesundheitsgerät verknüpft ist.

6. Verfahren nach Anspruch 1, wobei das Empfangen der Tag-Daten umfasst:
Erzeugen einer Nachricht; und
Senden der erzeugten Nachricht an einen Server, der betrieben werden kann, um eine oder mehrere Nachrichten basierend auf der erzeugten Nachricht an ein oder mehrere Gesundheitsgeräte zu senden.

7. Gesundheitsgerät (120, 130), das dazu ausgelegt ist:
eine erste Benutzerschnittstelle darzustellen, die dazu ausgelegt ist, einem Patienten in einer Gesundheitsumgebung zu ermöglichen, eine Patientenanfrage einzugeben;
Tag-Daten von einem Benutzer-Tag (110) zu empfangen, das mit einem ausgewählten Gesundheitsdienstleister in der Gesundheitsumgebung verknüpft ist, umfassend einen Gesundheitsdienstleisteridentifikator;
einen Abschnitt der Tag-Daten an einen Authentifizierungsserver (140) zu senden;
einen Zugangstoken von dem Authentifizierungsserver basierend auf der Authentifizierung des Abschnitts der Tag-Daten zu empfangen; und
eine zweite Benutzerschnittstelle auf dem Gesundheitsgerät darzustellen, die sich von der ersten Benutzerschnittstelle unterscheidet, wobei die zweite Benutzerschnittstelle zur Verwendung durch den ausgewählten Gesundheitsdienstleister basierend auf dem Zugangstoken und dem Gesundheitsdienstleisteridentifikator ausgelegt ist, um den ausgewählten Gesundheitsdienstleister der Patientenanfrage zu benachrichtigen.

8. Gesundheitsgerät nach Anspruch 7, wobei die erste Benutzerschnittstelle des mindestens einen ausgewählten Gesundheitsgeräts Funktionalitäten zur Verwendung durch den Patienten aufweist, wobei die zweite Benutzerschnittstelle des ausgewählten Gesundheitsgeräts Funktionalitäten zur Verwendung durch den Gesundheitsdienstleister aufweist, und wobei die zweite Benutzerschnittstelle und die Gesundheitsdienstleisterfunktionalitäten des ausgewählten Gesundheitsgeräts nicht über die erste Benutzerschnittstelle zugänglich sind.

9. Gesundheitsgerät nach Anspruch 7, wobei die Tag-Daten ferner einen Tag-Identifikator umfassen, und wobei der Zugangstoken von dem Authentifizierungsserver empfangen wird basierend auf einem Vergleich des Tag-Identifikators und des Gesundheitsdienstleisteridentifikators mit Authentifizierungsdaten, die auf dem Authentifizierungsserver gespeichert sind.

10. Gesundheitsgerät nach Anspruch 7, wobei das Gesundheitsgerät dazu ausgelegt ist:
Sitzungsdaten basierend auf den empfangenen Tag-Daten zu erzeugen und Aktionen über die zweite Benutzerschnittstelle durchzuführen; und
die Sitzungsdaten an den Authentifizierungsserver zu kommunizieren.

11. Gesundheitsgerät nach Anspruch 7, wobei das Erhalten der Tag-Daten von dem Benutzer-Tag automatisch über Nahfeldkommunikation ohne Benutzerinteraktion erfolgt, wenn das Benutzer-Tag in einem Bereich der Betriebsfähigkeit eines Tag-Lesegeräts vorhanden ist, das mit dem Gesundheitsgerät verknüpft ist.

12. Gesundheitsgerät nach Anspruch 7, wobei das Gesundheitsgerät dazu ausgelegt ist:
eine Nachricht zu erzeugen; und
die Nachricht an den Authentifizierungsserver zu senden, wobei der Authentifizierungsserver dazu ausgelegt ist, eine oder mehrere Nachrichten an ein oder mehrere Gesundheitsgeräte zu senden.

13. Gesundheitsgerät nach Anspruch 7, wobei der Benutzer-Tag dazu ausgelegt ist, von dem ausgewählten Gesundheitsdienstleister, der mit dem Benutzer-Tag verknüpft ist, getragen zu werden.

14. System, umfassend das Gesundheitsgerät (120, 130) nach Anspruch 7, das Benutzer-Tag (110) und den Authentifizierungsserver (140).

15. System nach Anspruch 14, wobei das System ein erstes Gesundheitsgerät und ein zweites Gesundheitsgerät umfasst, und wobei bei dem zweiten Gesundheitsgerät die erste Benutzerschnittstelle eine gesperrte Benutzerschnittstelle ist, die zweite Benutzerschnittstelle Funktionalitäten zur Verwendung durch den Gesundheitsdienstleister aufweist, und die zweite Benutzerschnittstelle und die Gesundheitsdienstleisterfunktionalitäten nicht über die erste Benutzerschnittstelle zugänglich sind.

## Revendications

1. Procédé de personnalisation d'un dispositif de soins de santé (120, 130), le procédé consistant en ce que le dispositif de soins de santé (120, 130) réalise les étapes suivantes :
présenter une première interface utilisateur sur le dispositif de soins de santé configurée pour permettre qu'un patient dans un environnement de soins de santé entre une demande de patient ;
recevoir des données d'étiquette en provenance d'une étiquette (110) d'utilisateur associée à un fournisseur de soins de santé sélectionné dans l'environnement de soins de santé comprenant un identifiant de fournisseur de soins de santé ;
envoyer une partie des données d'étiquette à un serveur d'authentification (140) ;
recevoir un jeton d'accès en provenance du serveur d'authentification sur la base de l'authentification de la partie des données d'étiquette ; et
présenter une seconde interface utilisateur sur le dispositif de soins de santé différente de la première interface utilisateur, la seconde interface utilisateur étant configurée pour être utilisée par le fournisseur de soins de santé sélectionné sur la base du jeton d'accès et de l'identifiant de fournisseur de soins de santé afin de notifier le fournisseur de soins de santé sélectionné de la demande du patient.

2. Procédé selon la revendication 1, dans lequel les données d'étiquette sont reçues en provenance de l'étiquette d'utilisateur par le biais d'une communication en champ proche.

3. Procédé selon la revendication 1, consistant en outre à :
recevoir une fermeture de session par l'intermédiaire de la seconde interface utilisateur ; et
présenter la première interface utilisateur à la place de la seconde interface utilisateur.

4. Procédé selon la revendication 1, dans lequel la première interface utilisateur présente des fonctionnalités pour une utilisation par un patient, dans lequel la seconde interface utilisateur présente des fonctionnalités pour une utilisation par un fournisseur de soins de santé, et dans lequel la seconde interface utilisateur et les fonctionnalités pour le fournisseur de soins de santé ne sont pas accessibles par l'intermédiaire de la première interface utilisateur.

5. Procédé selon la revendication 1, dans lequel la réception de données d'étiquette se produit automatiquement sans interaction de l'utilisateur lorsqu'une étiquette est présente dans une plage utilisable d'un lecteur d'étiquette associé au dispositif de soins de santé.

6. Procédé selon la revendication 1, dans lequel la réception des données d'étiquette consiste à :
générer un message ; et
émettre le message généré vers un serveur exploitable pour envoyer un ou plusieurs messages, sur la base du message généré, à un ou plusieurs dispositifs de soins de santé.

7. Dispositif de soins de santé (120, 130) configuré pour :
présenter une première interface utilisateur configurée pour permettre qu'un patient dans un environnement de soins de santé entre une demande de patient ;
recevoir des données d'étiquette en provenance d'une étiquette (110) d'utilisateur associée à un fournisseur de soins de santé sélectionné dans l'environnement de soins de santé comprenant un identifiant de fournisseur de soins de santé ;
envoyer une partie des données d'étiquette à un serveur d'authentification (140) ;
recevoir un jeton d'accès en provenance du serveur d'authentification sur la base de l'authentification de la partie des données d'étiquette ; et
présenter une seconde interface utilisateur sur le dispositif de soins de santé différente de la première interface utilisateur, la seconde interface utilisateur étant configurée pour être utilisée par le fournisseur de soins de santé sélectionné sur la base du jeton d'accès et de l'identifiant de fournisseur de soins de santé afin de notifier le fournisseur de soins de santé sélectionné de la demande du patient.

8. Dispositif de soins de santé selon la revendication 7, dans lequel la première interface utilisateur d'au moins un dispositif de soins de santé sélectionné présente des fonctionnalités pour une utilisation par un patient, dans lequel la seconde interface utilisateur du dispositif de soins de santé sélectionné présente des fonctionnalités pour une utilisation par un fournisseur de soins de santé, et dans lequel la seconde interface utilisateur et les fonctionnalités pour le fournisseur de soins de santé du dispositif de soins de santé sélectionné ne sont pas accessibles par l'intermédiaire de la première interface utilisateur.

9. Dispositif de soins de santé selon la revendication 7, dans lequel les données d'étiquette comprennent en outre un identifiant d'étiquette, et dans lequel le jeton d'accès est reçu en provenance du serveur d'authentification sur la base d'une comparaison de l'identifiant d'étiquette et de l'identifiant du fournisseur de soins de santé par rapport à des données d'authentification mémorisées sur le serveur d'authentification.

10. Dispositif de soins de santé selon la revendication 7, dans lequel le dispositif de soins de santé est configuré pour :
générer des données de session sur la base des données d'étiquette reçues et des actions réalisées par l'intermédiaire de la seconde interface utilisateur ; et
communiquer les données de session au serveur d'authentification.

11. Dispositif de soins de santé selon la revendication 7, dans lequel l'obtention des données d'étiquette en provenance de l'étiquette d'utilisateur se produit automatiquement par l'intermédiaire d'une communication en champ proche sans interaction de l'utilisateur lorsque l'étiquette d'utilisateur est présente dans une plage utilisable d'un lecteur d'étiquette associé au dispositif de soins de santé.

12. Dispositif de soins de santé selon la revendication 7, le dispositif de soins de santé étant configuré pour :
générer un message ; et
transmettre le message au serveur d'authentification, le serveur d'authentification étant configuré pour envoyer un ou plusieurs messages à un ou plusieurs dispositifs de soins de santé.

13. Dispositif de soins de santé selon la revendication 7, dans lequel l'étiquette d'utilisateur est configurée pour être portée par le fournisseur de soins de santé sélectionné associé à l'étiquette d'utilisateur.

14. Système comprenant le dispositif de soins de santé (120, 130) selon la revendication 7, l'étiquette d'utilisateur (110) et le serveur d'authentification (140) .

15. Système selon la revendication 14, le système comprenant un premier dispositif de soins de santé et un second dispositif de soins de santé, et dans lequel, pour le second dispositif de soins de santé, la première interface utilisateur est une interface verrouillée, la seconde interface utilisateur présente des fonctionnalités pour une utilisation par le fournisseur de soins de santé, et la seconde interface utilisateur et les fonctionnalités pour le fournisseur de soins de santé ne sont pas accessibles par l'intermédiaire de la première interface utilisateur.
